# EUROPEAN PATENT APPLICATION

(11) **EP 2 168 628 A1**
(43) Date of publication of application: **31.03.2010**
(21) Application number: 09160307.6
(22) Date of filing: 14.05.2009
(51) Int. Cl.: A61M 25/06, A61M 25/00

(54) **Safety I.V. catheter with a rotator and inbuilt plug to prevent blood back flow**

(30) Priority: 24.09.2008 IN KO16362008
(71) Applicant: Eastern Medikit Ltd., Kolkatta 700 032 (IN)
(72) Inventor: Sanjeev, Paul, 110 007 Delhi (IN); Raj, Narang Karun, 110 007 Delhi (IN)
(74) Representative: Harrison, Robert John

(57) **Abstract**

This invention relates to a medical safety intravenous (LV.) catheter (2) with retractable needle (3) comprising an inbuilt device to prevent blood back flow and the body of the I.V. Catheter is fitted with a rotator (4) in a shape of a tap with a rubber plug (6) fitted inside the rotator and the needle punches the rubber plug and makes way to pass through and after cannulation the needle is withdrawn and elastic nature of the rubber plug blocks the punctured hole and the rotator is to be rotated in a clockwise/ anti clockwise direction to bring in the holes in the lower portion of the rotator body with the opening in the body of the catheter making passage for infusion and after infusion, the tap is manipulated to close the holes and the needle tip forming gets locked in the locking insert (11).

## Description

The following specification particularly describes the nature of this invention and the manner in which it is to be performed:
This invention relates to a I.V. Catheter and, in particular, to a Safety I.V. Catheter with a Rotator and Inbuilt Plug to Prevent Blood Back Flow. In particular, the Safety intravenous catheter device for the invention has been so designed to prevent back flow of blood after cannulation at the time of withdrawal of the needle.

### BACKGROUND ART

It is well known that when cannulation is done i.e. when needle is inserted in the vein of a patient, blood comes out into the plastic tubing in which the needle is fitted. The purpose of cannulation is to leave the plastic tubing inside the vein and the needle is withdrawn and disposed off. In this process, blood rushes into the tube and body of the I.V. Catheter and even comes out.
To prevent this, paramedical staff presses the vein to avoid rush of blood. This method is too primitive, uncertain and ineffective in many cases.

### OBJECTS OF THE INVENTION

The purpose of this invention is to provide a Safety I.V. Catheter which would by manual Operation control and prevent such rush of blood after cannulation and be a Safety I.V. Catheter as well.

### SUMMARY OF THE INVENTION

Thus according to the basic aspect of the present invention there is provided an intravenous (I.V.) Catheter (with a Rotator and Inbuilt Plug to Prevent Blood Back Flow).

The I.V. Catheter comprises of a body, a needle hub capable of holding the needle in a manner that the non sharp end of the needle is fitted inside the needle hub and sharp end is used for cannulation. The body of the I.V. Catheter is fitted with a rotator in a shape of a tap with a rubber plug fitted inside the rotator. The needle punches the rubber plug and makes way to pass through. The details of the invention, its objects and advantages are explained hereunder in greater detail in relation to non-limiting exemplary illustration as per the following accompanying figures:

### BRIEF DESCRIPTION OF THE ACCOMPANYING FIGURES

Figure 1 illustrates an embodiment of the intravenous (I.V.) Catheter with rotator.

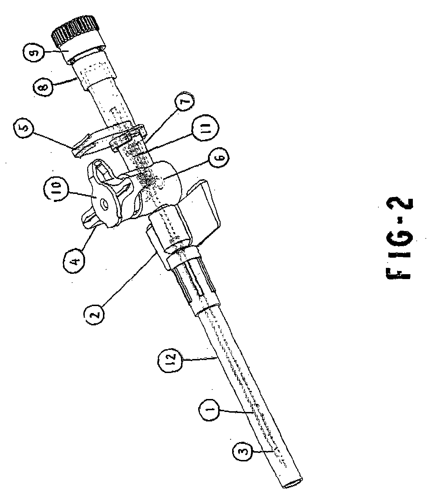

Figure 2 is the complete intravenous (I.V.) Catheter wherein the individual parts have been numbered as below:
   1. Portion marked as 1 is plastic tubing.
   2. Portion marked as 2 is body of the I.V. Catheter.
   3. Portion marked as 3 is needle.
   4. Portion marked as 4 is rotator.
   5. Portion marked as 5 is needle hub.
   6. Portion marked as 6 is rubber plug.
   7. Portion marked as 7 is the rotator holder.
   8. Portion marked as 8 is hub cover.
   9. Portion marked as 9 a leur lock.
   10. Portion marked as 10 is a cap to close the injection portion given in the rotator.
   11. Portion marked as 11 is tip protective housing.
   12. Portion marked as 12 is needle cover.

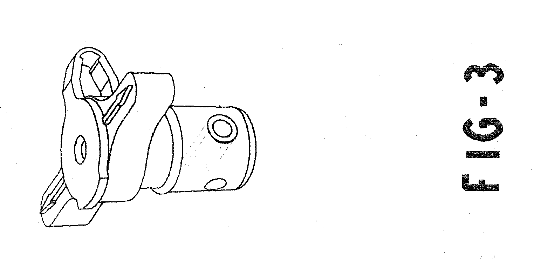

Figure 3 is an illustration of rotator with rubber plug.

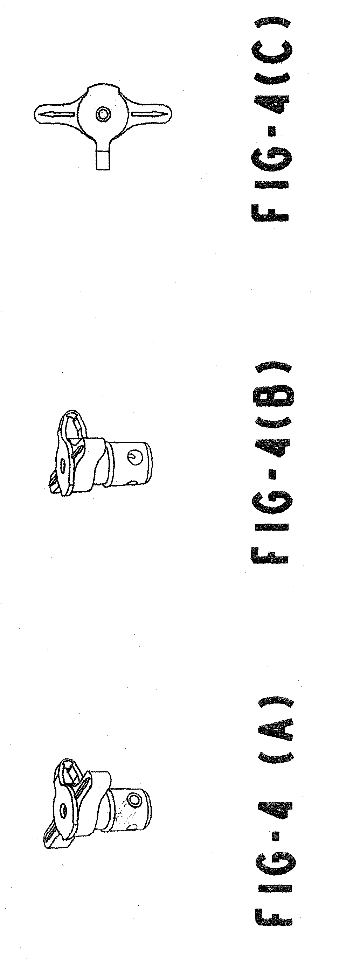

Figure 4 is an illustration of close up of rotator fitted with rubber plug having Figure 4(A) depicting the holes on the lower portion of rotator holder. Figure 4(B) depicts the plastic tunnel fitted inside the rubber plug. Figure 4(C) depicts the indicator on the top of the rotator plug.

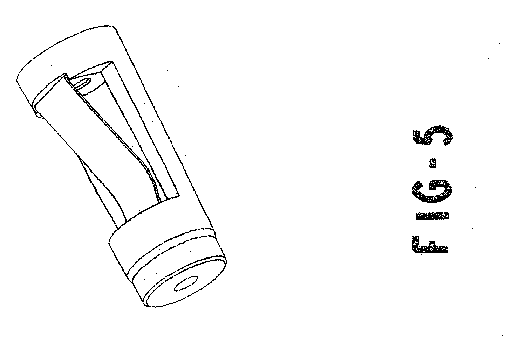

Figure 5 is illustration of tip protection housing which is the Safety feature.

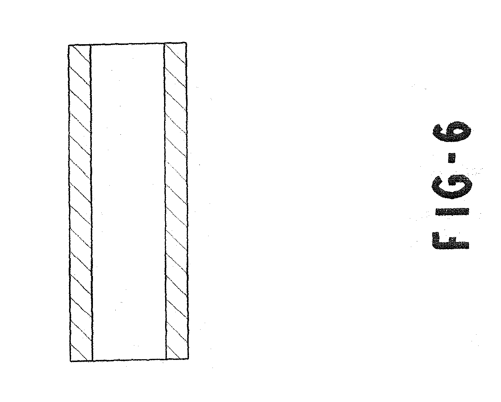

Figure 6 is illustration of the rubber plug.

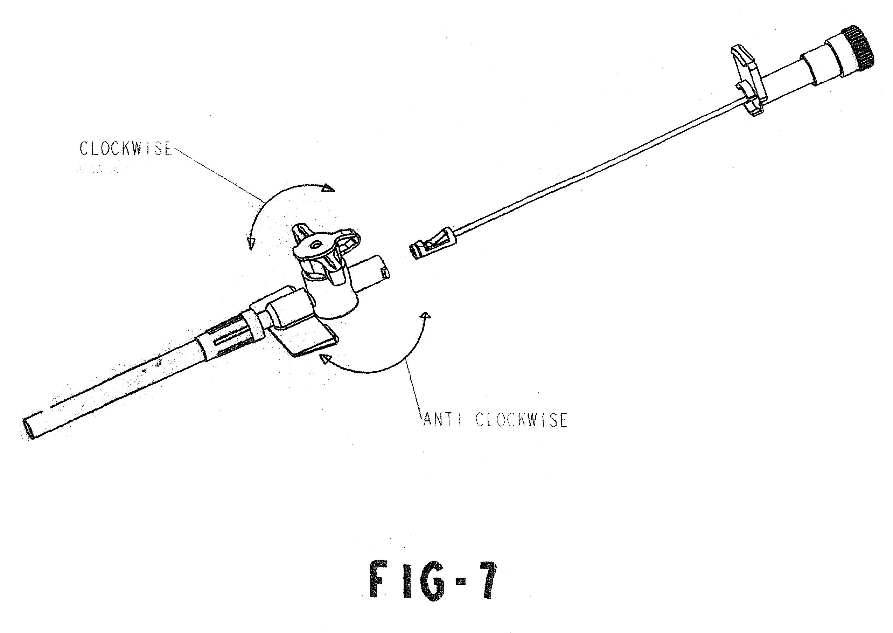

Figure 7 is illustration of needle withdrawn and rotator plug moved clockwise and anti clockwise to close the holes on the lower body of the rotator, simultaneously, the needle tip is locked in the tip protector housing making convenient disposal.

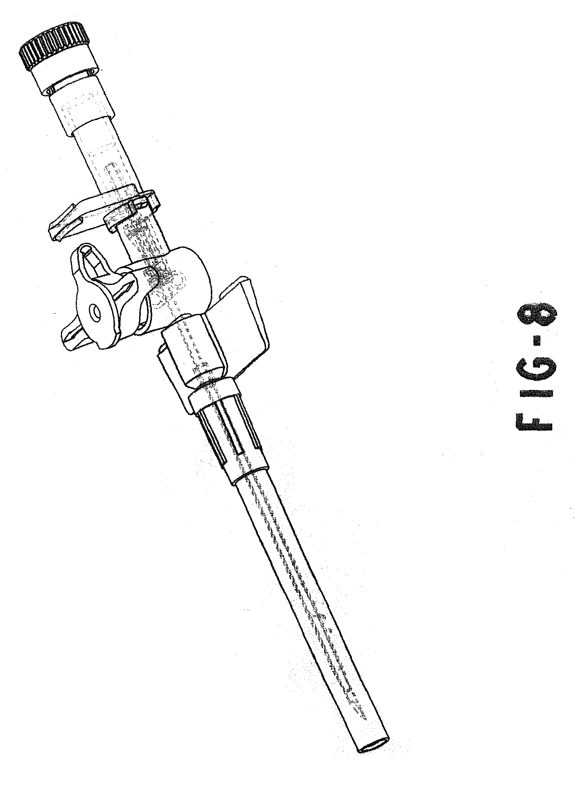

Figure 8 is the figure which shows the needle passing through the rubber plug by puncturing the rubber and then going into the plastic tubing. The rubber plug is fitted inside the rotator. The tip also passes through the tip protective housing which is the Safety Feature.

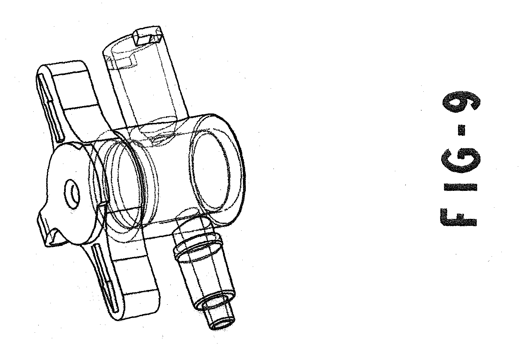

Figure 9 is illustration of rotator.

The rotator has a cylindrical body and a rubber plug in the hollow. The lower portion has a tunnel by way of plastic tubing fitted inside the rubber plug.

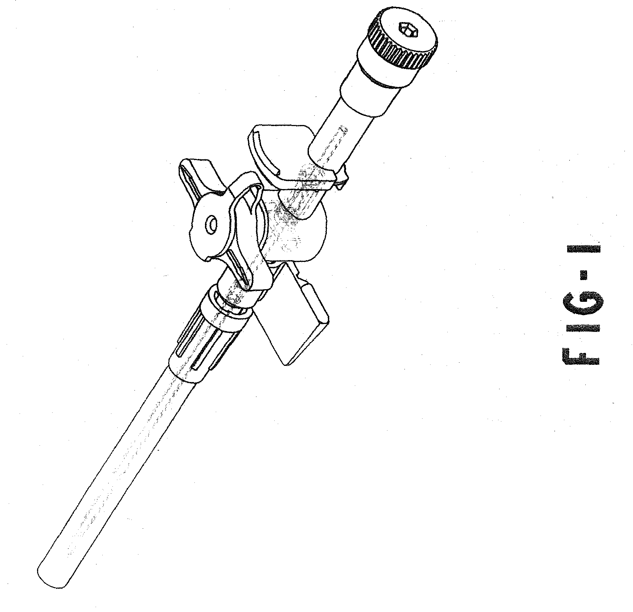

Reference is first invited to accompanying figure 1, which shows the intravenous I.V. Catheter device in accordance with the present invention wherein the product I.V. Catheter has a rotator with inbuilt plug (not shown).

The method of application and use of the above safety intravenous (I.V.) Catheter of the invention is further detailed hereunder:-
In use of the catheter device, initially the site of the catheter insertion on the patient's body is cleaned using alcohol wipes. Thereafter, the skin is punctured by using the needle tip which is inside a thin plastic tube. The needle tip is moved forward to puncture the vein. Immediately blood flash back is noticed in the blood collection chamber.

In the present invention, the blood is unable to come out from the body and spillage is avoided. This is an added safety feature making the user safe from the blood of the patient. Now, the fluid is administered. The fluid is administered through the body of the cannula. The needle is passing through the punctured hole made in the rubber stopper. The needle punctures the rubber stopper and makes its way through the plastic tubing. When the needle is pulled out after cannulation, the punctured hole is corresponding to the diameter of the needle. Due to the elastic nature of the rubber, the stopper closes the hole created forcefully by the needle. This prevents the back flow of the blood.

The rubber stopper is fitted in the hollow part of the body of the rotator. The rotator is to be rotated in clockwise/ anti clockwise direction. The purpose is to bring in line, the holes in the lower portion of the rotator body with the opening in the body of the cannula making passage for infusion. In such a case, the plastic tunnel fitted inside the plug comes in symmetry to the hole making passage for infusion of tunnels. After infusion, the tap is again manipulated to close the holes by disturbing the alignment with the holes and the opening i.e. rubber stopper comes in front of the opening.

It is now relevant to highlight the safety feature of the invention. Subsequently, the needle withdrawing action is initiated by withdrawing the needle out from the cannula and leaving the plastic tubing inside the body. The butterfly portion of the catheter remains outside for giving medication. In this process, the needle passes through the insert and its tip gets locked in the Locking Insert. In this position, the needle run its full length between the Locking Insert and the needle hub. The needle tip then gets locked inside the Locking Insert by means of stainless steel clip. The needle tip is locked in the manner that the tip cannot come out of the bend of the stainless steel clip.

As would be apparent from the above, the intravenous (I.V.) safety catheter thus achieves the locking of the needle tip within the Locking Insert free of any exposure to avoid needle stick injury and / or any spread of hospital acquired infection by way of unwanted needle contamination. Finally, by way of the above safe and convenient locking of the needle within Locking Insert of the catheter system of the invention, it is possible to safely discard the needle with Locking Insert in the sharp container.

Advantageously, the above (I.V.) Catheter is adapted to function as a safe device to protect the user against needle stick injuries. The above safety catheter device involving the Locking Insert can be manufactured out of simple materials including polypropylene, low density Polyethylene, stainless steel, acronytrle butadiene styrene, Silicon rubber, Floropolymer/ Polyurathene.

The Locking Insert, needle hub, flash back, needle hub are made of polypropylene. The needle and clip are made of stainless steel and pot cap is made of low density polyethylene.

The one way valve can be obtained of Silicon tube and adapted to facilitate extra medication and prevent back flow. The valve is specifically adapted to incorporate a recessed plug with protective skirt to effectively prevent contamination when the valve is not in use. Advantageously also pot cap is adapted for resting the finger to facilitate effective two-point grip for cannulation. The System offers safe and convenient needle less method for atraumatic administration of medicines. Moreover, it is possible to provide colour coded pot cap for gauge size identification. Angled and groove wings can be provided to offer easy fixation and prevent positioning and rolling of cannula over the patient's body. The catheter device so introduced to this invention is user friendly and provides a safe and simple catheter device for doctors and paramedical staff.

## Claims

1. An intravenous (I.V.) Catheter device with retractable needle comprising:
An inbuilt device to prevent blood back flow and where the body of the I.V. Catheter is fitted with a rotator in a shape of a tap with a rubber plug fitted inside the rotator and the needle punches the rubber plug and makes way to pass through and after cannulation the needle is withdrawn and elastic nature of the rubber plug blocks the punctured hole and the rotator is to be rotated in clockwise/ anti clockwise direction to bring in line the holes in the lower portion of the rotator body with the opening in the body of the catheter making passage for infusion and after infusion, the tap is manipulated to close the holes and where a needle different from others where a forming is provided at a distance from the tip, where the Locking Insert slides to the butterfly portion and the needle runs its length between the Locking Insert and the butterfly portion and the needle tip forming gets locked in the Locking Insert by means of a stainless steel clip provided inside the Tip Locking Insert and the Tip Locking Insert comes out of the butterfly portion fixed on the needle tip.

2. An intravenous (I.V.) Catheter device as claimed in claim 1 wherein the I.V. Catheter has a tap with a hollow place for a rubber plug.

3. An intravenous (I.V.) catheter as claimed in claim 1 wherein the body of the tap has holes at the bottom.

4. An intravenous (I.V.) catheter as claimed in claim 1 wherein the rubber plug has a plastic tunnel fitted inside the rubber plug.

5. An intravenous (I. V.) Catheter device as claimed in claim 1 wherein the needle hub is accompanied with a Locking Insert.

6. An intravenous catheter as claimed in claim 1 wherein the Locking Insert is fitted between the needle hub and the butterfly.

7. The intravenous (I.V.) catheter as claimed in claim 1 wherein Locking Insert has a stainless strip.

8. An intravenous (I.V.) Catheter device with needle substantially as herein described and illustrated with reference to the accompanying figures.

## Amended claims

### Amended claims in accordance with Rule 137(2) EPC.

**1.** An intravenous (I.V.) Catheter device with retractable needle (3) comprising:
An inbuilt device to prevent blood back flow and where the body of the I.V. Catheter is fitted with a rotator tap (4) with a rubber plug (6) fitted inside the rotator tap (4) and the needle (3) punctures the rubber plug (6) and makes way to pass through and after cannulation the needle (3) is withdrawn and an elastic nature of the rubber plug (6) blocks a punctured hole of the rubber plug (6) and the rotator tap (4) is to be rotated in clockwise/anti clockwise direction to bring in line holes in a lower portion of the rotator body with the opening in the body of the catheter (2) making a passage for infusion and after infusion, the rotator tap (4) is manipulated to close the holes and where a needle different from others where a forming is provided at a distance from the tip, where a locking insert slides to a butterfly portion of the intravenous (I.V.) Catheter and the needle (3) runs its length between the locking insert and the butterfly portion of the intravenous (I.V.) Catheter and the needle tip forming gets locked in the locking insert by means of a clip provided inside the tip locking insert and the tip locking insert comes out of the butterfly portion of the intravenous (I.V.) Catheter fixed on the needle (3) tip.

**2.** The intravenous (I.V.) Catheter device as claimed in claim 1, wherein the I.V. Catheter has a rotator tap (4) with a hollow place for the rubber plug (6).

**3.** The intravenous (I.V.) catheter as claimed in claim 1, wherein the body of the rotator tap (6) has holes at the bottom.

**4.** The intravenous (I.V.) catheter as claimed in claim 1, wherein the rubber plug (6) has a plastic tunnel fitted inside the rubber plug (6).

**5.** The intravenous (I. V.) Catheter device as claimed in claim 1, wherein a needle hub (5) is accompanied with the locking insert.

**6.** The intravenous catheter (I. V.) as claimed in claim 5, wherein the tip locking insert is fitted between the needle hub (5) and the butterfly portion of the intravenous (I.V.) Catheter.

**7.** The intravenous (I.V.) catheter as claimed in claim 1, wherein the tip locking insert comprises one of polypropylene, stainless steel, acronytryle butadiene styrene, silicon rubber and floropolymer/polyurathene.

**8.** The intravenous (I.V.) catheter as claimed in claim 5, wherein the needle hub (5) comprises polypropylene.

**9.** The intravenous (I.V.) catheter as claimed in claim 1, wherein the needle (3) comprises stainless steel.

**10.** The intravenous (I.V.) catheter as claimed in claim 1, wherein the tip locking insert comprises a stainless steel clip.
